# EUROPEAN PATENT APPLICATION

(11) **EP 3 572 512 A1**
(43) Date of publication of application: **27.11.2019**
(21) Application number: 18174066.3
(22) Date of filing: 24.05.2018
(51) Int. Cl.: C12N 15/10, C12N 15/63

(54) **A METHOD FOR ENGINEERING A PROTEIN**

(71) Applicant: B.R.A.I.N. AG, 64673 Zwingenberg (DE)
(72) Inventor: PELZER, Alexander, 64404 Bickenbach (DE); PUL, Ümit, 47259 Duisburg (DE)
(74) Representative: Fabry, Bernd

(57) **Abstract**

The present invention relates to a method for engineering a protein in a host cell, comprising the following steps:
identifying a protein of interest and introducing the coding sequence of the protein of interest into the genome of the host cell;
screening for hotspots for amino acid mutations in the protein of interest;
generating a set of specific guide RNAs and libraries of homologous recombination template that generate mutations at the desired sites within the protein coding region;
introducing the guide RNA and the library of homologous recombination template into the host cell, thereby producing mutated protein coding regions;
screening to select for cells that express the protein of interest with desired activity and/or property from the mutated protein coding regions, thereby providing an engineered protein.

## Description

### FIELD OF INVENTION

The invention relates to a method for engineering a protein in a host cell.

### STATE OF THE ART

Protein engineering has great significance for the improvement of enzymes in order to make them applicable for specific applications. Several strategies have been developed over the years. The predominant techniques for mutant library construction are either random mutagenesis or rational design. Random mutagenesis requires large screening capabilities as well as fast and reliable assay systems. Rational design requires detailed knowledge about the enzyme's structure and function in order to create smaller libraries.

As the number of solved protein structures increases and the tools for bioinformatic analysis of sequence databases and structure prediction improves, the focus more and more lies on the construction of small, high quality libraries (Pavelka, Antonin; Chovancova, Eva; Damborsky, Jiri (2009): HotSpot Wizard. A web server for identification of hot spots in protein engineering. In: Nucleic acids research 37 (Web Server issue), W376-83*;* Joosten HJ, Han Y, Niu W, Vervoort J, Dunaway-Mariano D, Schaap PJ. (2008). Identification of fungal oxaloacetate hydrolyase within the isocitrate lyase/PEPmutase enzyme superfamily using a sequence marker-based method. In: Proteins 70:157-166*;* Kuipers RKP, Joosten HJ, Verwiel E, Paans S, AkerboomJ, van der OostJ, Leferink NG,van Berkel WJ, Vriend G, Schaap PJ. (2009). Correlated mutation analyses on super-family alignments reveal functionally important residues. In: Proteins 76:608-616*).* The methods to generate these focused libraries can be categorized in either sequence-based or structure-based approaches that lead to identification of amino acid hotspots for mutagenesis (Wang, Chenghua; Huang, Ribo; He, Bingfang; Du, Qishi (2012): Improving the thermostability of alpha-amylase by combinatorial coevolving-site saturation mutagenesis. In: BMC bioinformatics 13: 263).

During enzyme engineering and production, the choice for a suitable production host is essential. The expression of correctly folded and functional protein highly depends on the selected expression host. In addition, host abilities like capability for protein secretion and posttranslational modifications have to be taken into account. Performing protein engineering experiments in a laboratory expression strain and subsequently changing the host for production can lead to the point where a significant improvement of an enzyme is very cost-intensive and therefore no longer economical.

The stable integration of expression cassettes into the host genome is the preferred method for enzyme production in an industrial environment (Daly, Rachel; Hearn, Milton T. W. (2005): Expression of heterologous proteins in Pichia pastoris. A useful experimental tool in protein engineering and production. In: Journal of molecular recognition : JMR 18 (2):119-138*;* Ahmad, Mudassar; Hirz, Melanie; Pichler, Harald; Schwab, Helmut (2014): Protein expression in Pichia pastoris. Recent achievements and perspectives for heterologous protein production. In: Applied microbiology and biotechnology 98 (12):5301-5317.). However, in contrast to *S*. *cerevisiae,* where homologous recombination (HR) is the predominant DNA repair mechanism, non-homologous end joining (NHEJ) occurs frequently in some other hosts, for example in *Pichia pastoris,* which makes targeted genomic modification a highly challenging task. CRISPR-Cas9 technology has been adapted for genetic modification of *P. pastoris.* However, while the introduction of Cas9-assisted NHEJ-mediated mutations was very efficient, the directed modification of chromosomal DNA by homologous recombination required the knock-out *of ku70* gene *(*Weninger, Astrid; Hatzl, Anna-Maria; Schmid, Christian; Vogl, Thomas; Glieder, Anton (2016): Combinatorial optimization of CRISPR/Cas9 expression enables precision genome engineering in the methylotrophic yeast Pichia pastoris. In: Journal of Biotechnology 235: 139-149*;* Weninger Astrid; Fischer, E. Jasmin; Raschmanová, Hana; Kniely, Claudia; Vogl, Thomas; Glieder, Anton (2018): Expanding the CRISPR/Cas9 toolkit for Pichia pastoris with efficient donor integration and alternative resistance markers. In: Journal of Cellular Biochemistry (119): 3183-3198*).* In the present invention, the modification of chromosomal copies of gene-of-interests by homologous recombination was achieved in *P*. *pastoris* wildtype cells with homologous recombination efficiency of up to 100 %. Therefore, the provided methods and protocols do not require the generation of *ku70* and/or *ku80* knock-out strains. Ku-deficient strains are known to exhibit severe growth defects, hypersensitivity against DNA-damages and genome stability *(*Baumann, Peter; Cech, R. Thomas (2000): Protection of telomeres by the Ku protein in fission yeast. In: Mol Biol Cell 11(10):3265-75*;* Bertuch, A. Alison; Lundblad, Victoria (2003): The Ku Heterodimer Performs Separable Activities at Double-Strand Breaks and Chromosome Termini. In: Mol Cell Biol 23(22): 8202-8215*;* de Sena-Tomás, Carmen; Yu, Eun Young; Calzada, Arturo; Holloman, K. William; Lue, F. Neal; Pérez-Martín, José (2015): Fungal Ku prevents permanent cell cycle arrest by suppressing DNA damage signaling at telomeres. In: Nucleic Acids Research 43(4):2138-2151*)* and thus not suitable for use as production strains in industrial applications

Therefore, it was one object of the present invention to provide a method for protein engineering which overcomes the disadvantages of the prior art. Furthermore, it was an object of the present invention to provide a method for protein-engineering in which the protein engineering is performed directly in the designated production host, which enables a cost-efficient and economical improvement of proteins.

### DESCRIPTION OF THE INVENTION

This object is solved by claim 1 of the present invention, which is directed to a method for engineering a protein in a host cell, comprising the following steps: identifying a protein of interest and introducing the coding gene of the protein of interest into the genome of the host cells; screening for hotspots for amino acid mutations in the protein of interest; generating a set of specific guide RNAs and one or more libraries of homologous recombination templates that generate mutations at the desired sites within the protein coding region; introducing the guide RNA and the library of homologous recombination template into the host cell, thereby producing mutated protein coding regions; screening to select for cells that express the protein of interest with desired activity and/or property from the mutated protein coding regions, thereby providing an engineered protein.

In a preferred embodiment according to the invention the coding gene of the protein of interest is introduced into the genome of the host cells by meand of a CRISPR-Cas-system.

Surprisingly the inventors have found that the method of the present invention enables a successful integration of a coding gene of the protein of interest into the genome of wildtype host cells. In a preferred embodiment of the invention the integration of a coding gene of the protein of interest into the genome of wildtype host cells is mediated by a CRISPR-Cas-system. Furthermore, the method of the present invention enables a functional expression and a biochemical characterization of the protein of interest. Afterwards, a rational library was designed using a 3DM database in order to optimize specific characteristics of the protein of interest. Finally, the inventors have shown with the method of the present invention that a CRISPR-Cas-system can be used for site directed gene manipulation of chromosomal gene copies using a mix of short DNA-repair templates enabling protein engineering. A widely used programmable system to introduce double strand DNA breaks is CRISPR-Cas9 (clustered regularly interspaced short palindromic repeats/CRISPR associated protein 9) which was reported to mediate targeted genome engineering in various pro- and eukaryotic organisms (Sander, Jeffry D.; Joung, J. Keith (2014): CRISPR-Cas systems for editing, regulating and targeting genomes. In: Nature biotechnology 32 (4): 347-355). CRISPR-Cas9 is a naturally occurring defense mechanism in bacteria and depends on the Cas9 endonuclease. Cas9 is guided by a short RNA molecule which is complementary to the site where Cas9 introduces a double strand DNA break. This break is repaired by the endogenous repair machinery. By changing 20 nt of the guide RNA, CRISPR-Cas9 can be reprogrammed to target a desired locus. Preferably the CRISPR-Cas-system is a Type II CRISPR-Cas-system and Cas9 is used as an endonuclease. This will be explained in more detail below.

As used herein, the singular form "a," "an," and "the" include plural references unless the context clearly dictates otherwise. For example, the term "a protein of interest" may include a plurality of protein of interests including mixtures thereof. Thus, for example, reference to "coding gene," "the coding gene," or "a coding gene" also includes a plurality of coding genes; use of the term "a nucleic acid" optionally includes, as a practical matter, many copies of that nucleic acid molecule; the term "host cell" also includes a plurality of host cells.

As used herein, the term "gene" means a locatable region of genomic sequence corresponding to a unit of inheritance. A gene may include regulatory regions, such as promoters, enhancers, 5 '-untranslated regions, intron regions, exon regions, 3 '-untranslated regions, transcribed regions, and other functional sequence regions that may exist as native genes or transgenes in a plant or a mammalian genome. Depending upon the circumstances, the term "target gene" can refer to the full-length nucleotide sequence of a gene targeted for binding and/or cleavage or the nucleotide sequence of a portion of a gene targeted for binding and/or cleavage. A target gene can be an endogenous gene or a transgene.

As used herein, the term "homologous recombination" refers to the exchange of nucleotide sequences at a conserved region shared by two genomic loci or by a donor DNA and a target site. Homologous recombination includes symmetric homologous recombination and asymmetric homologous recombination. Asymmetric homologous recombination may also be referred to as unequal recombination.

As used herein, the terms "target sequence" or "target site" refer to a nucleotide sequence against which a guide RNA capable of hybridizing. A target sequence may be genic or non-genic. In some aspects, a target sequence provided herein comprises a genic region. In other aspects, a target sequence provided herein comprises an intergenic region. In yet another aspect, a target sequence provided herein comprises both a genic region and an intergenic region. In an aspect, a target sequence provided herein comprises a coding nucleic acid sequence. In another aspect, a target sequence provided herein comprises a non-coding nucleic acid sequence. In an aspect, a target sequence provided herein is located in a promoter. In another aspect, a target sequence provided herein comprises an enhancer sequence. In yet another aspect, a target sequence provided herein comprises both a coding nucleic acid sequence and a non-coding nucleic acid sequence. In one aspect, a target sequence provided herein is recognized and cleaved by a double-strand DNA break inducing agent, such as a system comprising a CRISPR enzyme and a guide RNA.

In some cases, the host cell is selected from the group consisting of: an archaeal cell, a bacterial cell, a eukaryotic cell, a eukaryotic single-cell organism, a somatic cell, a germ cell, a stem cell, a plant cell, an algal cell, an animal cell, in invertebrate cell, a vertebrate cell, a fish cell, a frog cell, a bird cell, a mammalian cell, a pig cell, a cow cell, a goat cell, a sheep cell, a rodent cell, a rat cell, a mouse cell, a non-human primate cell, and a human cell. In some cases, the cell is in vitro. In some cases, the cell is in vivo.

A "host cell," as used herein, denotes an in vivo or in vitro eukaryotic cell, a prokaryotic cell (e.g., bacterial or archaeal cell), or a cell from a multicellular organism (e.g., a cell line) cultured as a unicellular entity, which eukaryotic or prokaryotic cells can be, or have been, used as recipients for a nucleic acid, and include the progeny of the original cell which has been transformed by the nucleic acid. It is understood that the progeny of a single cell may not necessarily be completely identical in morphology or in genomic or total DNA complement as the original parent, due to natural, accidental, or deliberate mutation. [0089]

The term "stem cell" is used herein to refer to a cell (e.g., plant stem cell, vertebrate stem cell) that has the ability both to self-renew and to generate a differentiated cell type (see Morrison et al. (1997) Cell 88:287-298). In the context of cell ontogeny, the adjective "differentiated", or "differentiating" is a relative term. A "differentiated cell" is a cell that has progressed further down the developmental pathway than the cell it is being compared with. Thus, pluripotent stem cells (described below) can differentiate into lineage-restricted progenitor cells (e.g., mesodermal stem cells), which in turn can differentiate into cells that are further restricted (e.g., neuron progenitors), which can differentiate into end-stage cells

(i.e., terminally differentiated cells, e.g., neurons, cardiomyocytes, etc.), which play a characteristic role in a certain tissue type, and may or may not retain the capacity to proliferate further. Stem cells may be characterized by both the presence of specific markers (e.g., proteins, RNAs, etc.) and the absence of specific markers. Stem cells may also be identified by functional assays both in vitro and in vivo, particularly assays relating to the ability of stem cells to give rise to multiple differentiated progeny.

Stem cells of interest include pluripotent stem cells (PSCs). The term "pluripotent stem cell" or "PSC" is used herein to mean a stem cell capable of producing all cell types of the organism. Therefore, a PSC can give rise to cells of all germ layers of the organism (e.g., the endoderm, mesoderm, and ectoderm of a vertebrate). Pluripotent cells are capable of forming teratomas and of contributing to ectoderm, mesoderm, or endoderm tissues in a living organism. Pluripotent stem cells of plants are capable of giving rise to all cell types of the plant (e.g., cells of the root, stem, leaves, etc.).

PSCs of animals can be derived in a number of different ways. For example, embryonic stem cells (ESCs) are derived from the inner cell mass of an embryo whereas induced pluripotent stem cells (iPSCs) are derived from somatic cells (Takahashi et. al, Cell. 2007 Nov 30;131(5):861-72; Takahashi et. al, Nat Protoc. 2007;2(12):3081-9; Yu et. al, Science. 2007 Dec 21;318(5858):1917-20. Epub 2007 Nov 20). Because the term PSC refers to pluripotent stem cells regardless of their derivation, the term PSC encompasses the terms ESC and iPSC. PSCs may be in the form of an established cell line, they may be obtained directly from primary embryonic tissue, or they may be derived from a somatic cell. PSCs can be target cells of the methods described herein.

By "embryonic stem cell" (ESC) is meant a PSC that was isolated from an embryo, typically from the inner cell mass of the blastocyst. Stem cells of interest also include embryonic stem cells from other primates, such as Rhesus stem cells and marmoset stem cells. The stem cells may be obtained from any mammalian species, e.g. human, equine, bovine, porcine, canine, feline, rodent, e.g. mice, rats, hamster, primate, etc. (Thomson et al. (1998) Science 282:1145; Thomson et al. (1995) Proc. Natl. Acad. Sci USA 92:7844; Thomson et al. (1996) Biol. Reprod. 55:254; Shamblott et al., Proc. Natl. Acad. Sci. USA 95:13726, 1998). In culture, ESCs typically grow as flat colonies with large nucleo-cytoplasmic ratios, defined borders and prominent nucleoli. In addition, ESCs express SSEA-3, SSEA-4, TRA-1-60, TRA-1-81, and Alkaline Phosphatase, but not SSEA-1. Examples of methods of generating and characterizing ESCs may be found in, for example, US Patent No. 7,029,913 , US Patent No. 5,843,780 , and US Patent No. 6,200,806 . Methods for proliferating hESCs in the undifferentiated form are described in WO 99/20741, WO 01/51616 , and WO 03/020920 .

Human embryonic germ stem cells or human embryonic germ cells are not part of the invention. By "embryonic germ stem cell" (EGSC) or "embryonic germ cell" or "EG cell" is meant a PSC that is derived from germ cells and/or germ cell progenitors, e.g. primordial germ cells, i.e. those that would become sperm and eggs. Embryonic germ cells (EG cells) are thought to have properties similar to embryonic stem cells as described above. Examples of methods of generating and characterizing EG cells may be found in, for example, US Patent No. 7,153,684 ; Matsui, Y., et al., (1992) Cell 70:841; Shamblott, M., et al. (2001) Proc. Natl. Acad. Sci. USA 98: 113; Shamblott, M., et al. (1998) Proc. Natl. Acad. Sci. USA, 95:13726; and Koshimizu, U., et al. (1996) Development, 122:1235.

In one embodiment according to the invention the host cell is a prokaryotic host cell. In another embodiment according to the invention the prokaryotic host cell is selected from the group comprising *Bacillus, Escherichia coli, Streptomyces, Corynebacterium.*

In accordance with the present invention, the host cell can be a prokaryote cell. Such cells serve as a host for expression of recombinant proteins for production of recombinant therapeutic proteins of interest. Exemplary host cells include E. coli and other Enterobacteriaceae, Escherichia sp., Campylobacter sp., Wolinella sp., Desulfovibrio sp., Vibrio sp., Pseudomonas sp. Bacillus sp., Bacteroides sp., Listeria sp., Staphylococcus sp., Streptococcus sp., Peptostreptococcus sp., Megasphaera sp., Pectinatus sp., Selenomonas sp., Zymophilus sp., Actinomyces sp., Arthrobacter sp., Frankia sp., Micromonospora sp., Nocardia sp., Propionibacterium sp., Streptomyces sp., Lactobacillus sp., Lactococcus sp., Leuconostoc sp., Pediococcus sp., Acetobacterium sp., Agrobacterium sp., Aliivibrio sp., Eubacterium sp., Haloarcula sp., Halobacterium sp., Heliobacterium sp., Heliospirillum sp., Sporomusa sp., Spiroplasma sp., Ureaplasma sp., Erysipelothrix, sp., Corynebacterium sp. Enterococcus sp., Clostridium sp., Mycoplasma sp., Mycobacterium sp., Actinobacteria sp., Salmonella sp., Shigella sp., Moraxella sp., Helicobacter sp, Paracoccidioides sp., Stenotrophomonas sp., Micrococcus sp., Neisseria sp., Bdellovibrio sp., Hemophilus sp., Thermus sp., Klebsiella sp., Proteus sp., Enterobacter sp., Serratia sp., Citrobacter sp., Pseudomonas sp., Proteus sp., Rhodobacter sp., Rhodopseudomonas sp., Rhodospirillum sp., Serratia sp., Yersinia sp., Acinetobacter sp., Actinobacillus sp. Bordetella sp., Brucella sp., Capnocytophaga sp., Cardiobacterium sp., Eikenella sp., Francisella sp., Haemophilus sp., Kingella sp., Pasteurella sp., Flavobacterium sp. Xanthomonas sp., Burkholderia sp., Aeromonas sp., Plesiomonas sp., Legionella sp. and alpha-proteobacteria such as Wolbachia sp., Comamonas sp., Pyrobaculum sp., Sinorhizobium sp., cyanobacteria, spirochaetes, green sulfur and green non-sulfur bacteria, Gram-negative cocci, Gram negative bacilli. In a preferred embodiment according to the invention the prokaryotic host is *Bacillus subtilis.*

In another embodiment according to the invention the host cell is a eukaryotic host cell. In another embodiment according to the invention the eukaryotic host cell is selected from the group comprising *Pichia sp., Aspergillus sp" Kluyveromyces sp., Saccharomyces sp., Candida sp., Trichoderma sp.,Penicillium sp., Neurospora sp., Chrysosporium sp., Cladosporium sp., Phytophthora sp.,* Scytalidium *sp., more preferred Saccharomyces cerevisiae, Pichia pastoris, Aspergillus nidulans, Aspergillus niger, Trichoderma reseei, Klyveromyces lactis, Klyveromyces marxianus, Neurospora crassa.* In another embodiment eukaryotic host cell comprise plants, insects, molluscs, birds, fish and mammals.

In a most preferred embodiment according to the invention the host cell is *Pichia pastoris.* The methanol utilizing yeast *P. pastoris* is a widely used eukaryotic production host for the expression of recombinant protein, which is also known as *Komagataela phaffii.* Multiple reasons make this expression host attractive for industrial use. Its high cell density growth, the ability to use the tightly regulated and strong inducible *aox1* promotor as well as the high secretion capacity of recombinant proteins by these of the alpha mating factor from *S*. *cerevisae* (Cereghino, Joan Lin; Cregg, James M. (2000): Heterologous protein expression in the methylotrophic yeast Pichia pastoris. In: FEMS Microbiol Rev 24 (1): 45-66).

In one embodiment according to the invention the protein of interest may be any protein that is suitable for the method according to the invention. In another embodiment according to the invention the protein of interest is for example a hydrolase. Hydrolase is a class of enzyme that is commonly used as biochemical catalysts that utilize water to break a chemical bond. Some common examples of hydrolase enzymes are esterases including lipases, phosphatases, glycosidases, peptidases, and nucleosidases. Another example is amylase. In another embodiment according to the invention the protein of interest is an amylase. In a preferred embodiment according to the invention the amylase is an α-amylase. α-amylases are endo-amylases that play an important role in carbohydrate metabolism in microorganisms, plants, and higher organisms. They function as starch degrading enzymes which catalyze the hydrolysis of internal α-D-1,4-glycosidic linkages of amylose resulting in anomeric mono- or oligosaccharides. These enzymes are extensively used in broad industrial fields like starch liquefaction, detergents, baking, brewing, food, textile and paper industry. However, each industrial field requires specific α-amylase properties for optimal performance. These include substrate specificity, activity as well as pH- and thermostability under process relevant conditions (Asgher, M.; Asad, M. Javaid; Rahman, S. U.; Legge, R. L. (2007): A thermostable α-amylase from a moderately thermophilic Bacillus subtilis strain for starch processing. In: Journal of Food Engineering 79 (3), S. 950-955.).

The inventors have surprisingly found that it is possible with the method according to the invention to engineer a protein with rational enzyme design using CRISPR-Cas9 and a set of specific sgRNAs combined with a library of homologous recombination template by homologous recombination in Ku-proficient cells with an efficiency of almost 100 %. In a preferred embodiment according to the invention the α-amylase is LrAmy and/or RoAmy. With the method of the present invention it is, for example, possible to engineer a protein using a CRISPR-Cas-system on the basis of optimizing the two α-amylases LrAmy and RoAmy encoded from the genome of the designated production host *P. pastoris.* Furthermore a successful integration of amylase genes into the *aox1* locus mediated by CRISPR-Cas-system can be reached.

In another embodiment according to the invention the protein coding region is *aox1.*

### BRIEF DESCRIPTION OF THE DRAWINGS

**Fig. 1** shows the schematic overview of the CRISPR mediated integration and mutagenesis strategy. α-amylase expression cassettes are integrated into the genomic *aox1* locus.
**Fig. 2** shows the schematic overview of mutagenesis strategy by codon exchange via homologous recombination (HR). Scheme of short oligo nucleotides (90 nt) as HR-templates containing codons for desired amino acid exchange. HR-templates contain silent mutations in PAM and Cas9 seed region to inhibit the introduction of double-strand DNA break after homologous recombination.
**Fig. 3** shows agarose gels of colony PCRs for the analysis of the integration of the amylase gene cassettes. Correct integration is indicated by PCR products of 3.8 kbp in length matching the size of the expression cassette. PCR products of the wild type (WT) have a length of 2.2 kbp.
**Fig. 4** shows the SDS PAGE analysis of supernatants after α-amylase production by *P. pastoris.* Medium control lane shows the inherent enzyme from the expression medium. Wildtype (WT) and empty vector (EV) control additionally show endogenous proteins secreted by *P. pastoris.* Both α-amylase expression strains show proteins at expected molecular weights.
**Fig. 5** shows the pH profiles for LrAmy and RoAmy.
**Fig. 6** shows the temperature profiles for LrAmy and RoAmy.
**Fig. 7** shows the schematic overview of the integration strategy of a short donor DNA (100 nt) containing a *Psi*I recognition site. The successful integration can be easily analyzed by digestion of the DNA after amplification by PCR. *Psi*I recognition site does not occur in the native *aox1* gene. Furthermore, the *Psi*I site replaces the PAM sequence to inhibit a recleavage of the integration products by Cas9 after a successful HR.
**Fig. 8** shows the PCR products of the *aox1* locus from 5 different *P. pastoris* clones after HR-template integration. PCR products were digested by *Psi*I for verification of integration. All analyzed colonies showed successful integration. The correct integration was further confirmed by Sanger sequencing.
**Fig. 9** shows the identified hot spots and amino acid exchanges to improve the specific activity of LrAmy.
**Fig. 10** shows the identified hot spots and amino acid exchanges to improve the thermostability of RoAmy.
**Fig. 11** shows the specific activities of generated LrAmy mutants compared to wt LrAmy.
**Fig. 12** shows the relative stabilities and relative activities of generated RoAmy mutants compared to the wt RoAmy.

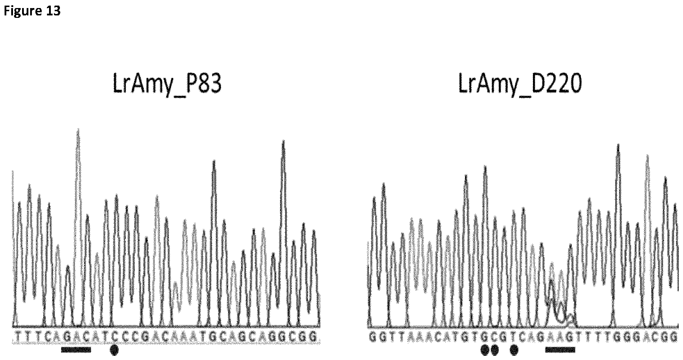

**Fig. 13** shows sequencing results of P83 in LrAmy as representative for a specific amino acid exchange and D220 in LrAmy as representative for an exchange with a degenerate codon. Mutations are highlighted. Exchanged codon is underlined (bar). For both sites, PAM was altered during mutagenesis and silent mutations were introduced (dots). The chromatogram for P83 shows clear signals at all mutated sites. Chromatogram of D220 shows overlapping signals where the mutagenesis codon is introduced. This indicates successful mutagenesis with degenerate codons. Silent mutation sites of D220 show clear signals without WT background.

### CRISPR-CAS

Clustered regularly interspersed short palindromic repeats (CRISPR) exist in many bacterial genomes and have been found to play an important role in adaptive bacterial immunity. Transcription of these arrays gives rise to CRISPR RNAs (crRNAs) that direct sequence-specific binding of CRISPR-Cas complexes to DNA targets in cells for gene repression or DNA cleavage. The specificity of these complexes allows novel *in vivo* applications for strain engineering.

In one embodiment according to the invention methods of rational, multiplexed manipulation of chromosomes within open reading frames or within multiple genes in any segment of a chromosome, in which various CRISPR systems are used are described. These methods provide more efficient combinatorial genome engineering than those previously available.

The methods comprise introducing components of the CRISPR system, including CRISPR-associated nuclease Cas9 and a sequence-specific guide RNA (gRNA) into host cells, resulting in sequence-directed double strand DNA breaks using the ability of the CRISPR system to induce such breaks. Components of the CRISPR system, including the CRISPR-associated nuclease Cas9 and a sequence-specific guide RNA (gRNA), can be introduced into cells encoded on one or more vector, such as a plasmid.

There are several different CRISPR-Cas systems and the nomenclature and classification of these have changed as the systems have been characterized. In particular, CRISPR-Cas systems have now been reclassified into two classes, containing six types and nineteen subtypes (Makarova et al., Nature Reviews Microbiology (2015) 13 : 1-15*;* Shmakov et al., Nature Reviews Microbiology (2017) 15: 169-182). This classification is based upon identifying all *cas* genes in a CRISPR-Cas locus and then determining the signature genes in each CRISPR-Cas locus, thereby determining whether the CRISPR-Cas systems should be placed in either Class 1 or Class 2 based upon the genes encoding the effector module, i.e., the proteins involved in the interference stage.

A CRISPR locus includes a number of short repeating sequences referred to as "repeats." Repeats can form hairpin structures and/or repeats can be unstructured single-stranded sequences. The repeats occur in clusters. Repeats frequently diverge between species. Repeats are regularly interspaced with unique intervening sequences, referred to as "spacers," resulting in a repeat-spacer-repeat locus architecture. Spacers are identical to or are homologous with known foreign invader sequences. A spacer-repeat unit encodes a cnsprRNA (crRNA). A crRNA refers to the mature form of the spacer-repeat unit. A crRNA contains a spacer sequence that is involved in targeting a target nucleic acid {e.g., possibly as a surveillance mechanism against foreign nucleic acid). A spacer sequence is typically located towards the 5'-end of a crRNA (e.g. in a Type I (e.g. Cascade) system; for a description of the Cascade complex see, e.g., Jore, M. M. et al, "Structural basis for CRISPR RNA-guided DNA recognition by Cascade," Nature Structural & Molecular Biology (2011) 18:529-536) or at the 3' end of the spacer of a crRNA in a Type II system (e.g., in a Type II CRISPR system, described more fully below), directly adjacent to the first stem.

Thus, crRNA has a region of complementarity to a potential DNA target sequence and a second region that forms base-pair hydrogen bonds with the tracrRNA to form a secondary structure, typically to form at least a stem structure. The tracrRNA and a crRNA interact through a number of base-pair hydrogen bonds to form secondary RNA structures. Complex formation between tracrRNA/crRNA and a Cas9 protein results in conformational change of the Cas protein that facilitates binding to DNA, endonuclease activities of the Cas9 protein, and crRNA-guided site-specific DNA cleavage by the endonuclease. For a Cas9 protein/tracrRNA/crRNA complex to cleave a DNA target sequence, the DNA target sequence is adjacent to a cognate protospacer adjacent motif (PAM).

By a "CRISPR-Cas system" as used herein, is meant any of the various CRISPR-Cas classes, types and subtypes. Currently two classes of CRISPR systems have been described, Class 1 and Class 2. Class 1 systems have a multi-subunit crRNA-effector complex, whereas Class 2 systems have a single protein, such as Cas9, Cpf1, CasX, CasY C2c1, C2c2, C2c3, or a crRNA-effector complex. Class 1 systems comprise Type I, Type III and Type IV systems. Class 2 systems comprise Type II, Type V and Type VI systems.

Type II systems include *cas1, cas2* and *cas9* genes. There are two strands of RNA in Type II systems, a CRISPR RNA (crRNA) and a transactivating CRISPR RNA (tracrRNA). The tracrRNA hybridizes to a complementary region of pre-crRNA causing maturation of the pre-crRNA to crRNA. The duplex formed by the tracrRNA and crRNA is recognized by, and associates with a multidomain protein, Cas9, encoded by the *cas9* gene, that combines the functions of the crRNA-effector complex with target DNA cleavage. Cas9 is directed to a target nucleic acid by a sequence of the crRNA that is complementary to, and hybridizes with, a sequence in the target nucleic acid.

It has been demonstrated that these minimal components of the RNA-based immune system can be reprogrammed to target DNA in a site-specific manner by using a single protein and two RNA guide sequences or a single RNA molecule. Type II systems are further divided into three subtypes, subtypes II-A, II-B and II-C.

crRNA biogenesis in a Type II CRISPR system comprises a tracrRNA. The tracrRNA is typically modified by endogenous RNaseIII. The tracrRNA hybridizes to a crRNA repeat in the pre-crRNA array. Endogenous RNaseIII is recruited to cleave the pre-crRNA. Cleaved crRNAs are subjected to exoribonuclease trimming to produce the mature crRNA form (e.g., 5' trimming). The tracrRNA typically remains hybridized to the crRNA. The tracrRNA and the crRNA associate with a site-directed polypeptide (e.g., Cas9). The crRNA of the crRNA-tracrRNA-Cas9 complex can guide the complex to a target nucleic acid to which the crRNA can hybridize. Hybridization of the crRNA to the target nucleic acid activates a wild-type, cognate Cas9 for target nucleic acid cleavage. Target nucleic acid in a Type II CRISPR system comprises a PAM. In some embodiments, a PAM is essential to facilitate binding of a site-directed polypeptide (e.g., Cas9) to a target nucleic acid.

Cas9 is an exemplary Type II CRISPR Cas protein and serves as an endonuclease. The mature crRNA that is base-paired to trans-activating crRNA (tracrRNA) forms a two-part RNA structure, also called "dual-guide," that directs the Cas9 to introduce double strand DNA breaks (DSBs) in target DNA. Cas9 can be programmed by the tracrRNA/crRNA to cleave, site-specifically, target DNA using two distinct endonuclease domains (HNH and RuvC/RNase H-like domains) (see U.S. Published Patent Application No. 2014-0068797, published 6 March 2014*;* see also Jinek M., et al, "A programmable dual-RNA-guided DNA endonuclease in adaptive bacterial immunity," Science (2012) 337:816-821*),* one for each strand of the DNA's double helix. RuvC and HNH together produce double-strand DNA breaks, and separately can produce single-strand DNA breaks. At sites complementary to the crRNA-guide (spacer) sequence, the Cas9 HNH nuclease domain cleaves the complementary strand and the Cas9 RuvC-like domain cleaves the non-complementary strand. Dual-crRNA/tracrRNA molecules have been engineered into single-chain crRNA/tracrRNA molecules. These single-chain crRNA/tracrRNA direct target sequence-specific Cas9 double-strand DNA cleavage.

A large number of Cas9 orthologs are known in the art as well as their associated tracrRNA and crRNA components. A number of orthogonal Cas9 proteins have been identified including Cas9 proteins from *Neisseria meningitidis, Streptococcus thermophilus and Staphylococcus aureus.*

As used herein, "a Cas protein" such as "a Cas9 protein," "a Cas3 protein," "a Cpfl protein," etc. refers to a Cas protein derived from any species, subspecies or strain of bacteria that encodes the Cas protein of interest, as well as variants and orthologs of the particular Cas protein in question. The Cas proteins can either be directly isolated and purified from bacteria, or synthetically or recombinantly produced, or can be delivered using a construct encoding the protein, including without limitation, naked DNA, plasmid DNA, a viral vector and mRNA for Cas expression. Non-limiting examples of Cas proteins include Cas1, Cas 1B, Cas2, Cas3, Cas4, Cas5, Cas6, Cas7, Cas8, Cas9 (also known as Csn1 and Csx12), Cpf1 (also known as Cas12a), CasX, CasY, Cas10, Cpf1, Csy1, Csy2, Csy3, Cse1, Cse2, Csc1, Csc2, Csa5, Csn2, Csm2, Csm3, Csm4, Csm5, Csm6, Cmrl, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, C2C1, C2C2, C2C3, homologs thereof, or modified versions thereof. These enzymes are known.

Variants and modifications of Cas9 proteins are known in the art, for example in U.S. Patent Publication 2014/0273226, published Sep 18, 2014, discusses the *S*. *pyogenes* Cas9 gene, Cas9 protein, and variants of the Cas9 protein including host-specific codon-optimized Cas9 coding sequences.

In a preferred embodiment according to the invention the CRISPR-Cas-system is a Class II system. In another preferred embodiment according to the invention Cas9 is used as an endonuclease.

### EXAMPLES

### Material & Methods

### Strains and Media

The strain *Pichia pastoris* CBS7435 Mut+ was used for heterologous expression of amylases. *Escherichia coli* DH10beta was ordered from New England Biolabs and used for routine cloning and propagation of plasmids. *E. coli* BL21(DE3) was ordered from New England Biolabs and used for production of GFP1-10 (Santos-Aberturas, Javier; Dörr, Mark; Waldo, Geoffrey S.; Bornscheuer, Uwe T. (2015): In-Depth High-Throughput Screening of Protein Engineering Libraries by Split-GFP Direct Crude Cell Extract Data Normalization. In: Chemistry & biology 22 (10):1406-1414. DOI: 10.1016/j.chembiol.2015.08.014). Cultivation of *E. coli was* performed in Luria-Bertani (LB) medium and on LB-agar plates containing corresponding antibiotics.

*P. pastoris* was cultivated at 30 °C in YPD medium (10 g/l yeast extract, 20 g/l bactopeptone, 2 % w/v dextrose) and on YPD agar plates containing 200 µg/ml G418. Expression of α-amylases was performed for 3 days in 96 well plates using the Media development M-KIT-500 (M2P-Labs) with the modifications that no thiamin-HCI was added to the vitamin mix solution and no micro element solution was used. 25 µl of YPD precultures with an OD₆₀₀ of 4 - 6 were inoculated in 500 µl expression medium per well. Glucose feed was induced by adding 1 % v/v enzyme mix twice every 10 and 16 h. Sterile methanol (0.5 %) was added once 16 h after inoculation and afterwards together with the enzyme mix to induce the *aox1* promotor. Supernatants were harvested after 3 days of fermentation.

### Cloning and Plasmid generation

Cloning of amylase genes was performed via Gibson Assembly using Gibson Assembly® Master Mix (NEB) according to the manufactural protocol. For standard PCR amplifications Phusion Flash Master Mix (NEB) was used with the PCR program: (98°C 10 sec [98°C 1sec, 55°C 5sec, 72°C 15 s/kb] x 30, 72°C 2 min). All used restriction endonucleases and ligases were ordered from NEB and used as described in manufacturer's protocol. α-amylase genes were ordered as DNA strings at GeneArt, fused to a GFP11 tag by overlap extension PCR and subcloned into pMA5 derivate (Dartois, V., Coppée, J. Y., Colson, C., & Baulard, A. (1994: Genetic analysis and overexpression of lipolytic activity in Bacillus subtilis. In: Applied and Environmental Microbiology, 60(5), 1670-1673.) by *Nde*I and *Hind*III sites. These plasmids were restriction digested with *Nde*I and *Hind*III*.*

The resulting 1311bp fragment containing the amylase genes were ligated into an equally opened pET26b(+) creating pET26b(+)*_lrAmy_gfp11* and pEJ2Sb(+)_*roAmy_gfp11.* LrAmy-GFP11 and RoAmy-GFP11 were amplified from pET26b(+)*_lrAmy_gfp11* using the primers Lra-Ins-FW (SEQ ID No. 1)/pPpT4-Ins_RV (SEQ ID No.2) and pET26b(+)_*roAmy_gfp11* using the primer Ror-Ins-FW (SEQ ID No. 3)/pPpT4-Ins_RV (SEQ ID No. 2). PCR products were cloned by Gibson cloning into pPpT4_alphaS (Zeo^{R}) after amplification of the plasmid using the primers Lra-Vec-RV (SEQ ID No. 4)/pPpT4-Vec_FW (SEQ ID No. 5) to yield pPpT4-alphaS-*lrAmy-gfp11* and the primers Ror-Vec-RV (SEQ ID No. 6)/pPpT4-Vec_FW (SEQ ID No. 5) to yield pPpT4-alphaS*-roAmy-gfp11.*

All Plasmids were isolated using GeneJET Plasmid Miniprep Kit (Thermo scientific) and sequenced at GATC Biotech. For targeted gene integration via CRISPR mediated HR, the integration cassettes of *alphaS-lrAmy-gfp11* and *alphaS-roAmy-gfp11* were amplified from pPpT4-alphaS-*lrAmy*-*gfp11* and pPpT4-alphaS-*roAmy-gfp11* with primers containing short homologous overhangs (70 - 90 bp) to *aox1* (Amy_lnt_FW (SEQ ID No. 7) and Amy_lnt_RV (SEQ ID No. 8)). The plasmid pPpT6e-*cas9* (pPpT4 derivative containing KanMX cassette and Cas9) was used for expression of *cas9* and sgRNAs. sgRNAs fused to Hammerhead-Ribozyme sequence were ordered as single strand oligonucleotides (Biomers). 5 µl of the complementary oligonucleotides (100 pmol/µl) were hybridized in the annealing buffer (10 mM Tris, 1 mM EDTA, 50 mM NaCl (pH 8.0)) in a total volume of 40 µl through heating at 98°C and letting it cool down at room temperature. The dsDNA contained corresponding restriction sites for cloning into pPpT6-*cas9*. pPpT6-*cas9*-sgRNA was linearized via digestion using *Bpi*I*,* mixed with hybridized gRNA, and ligated using T4-Quick-Ligase (NEB). The oligonucleotides for specific gene mutagenesis via homologous recombination were ordered and hybridized analogously.

### Transformation

Chemical competent cells of *E*. *coli* DH10beta (NEB) were transformed after standard protocol. Cells were thawed on ice and incubated with DNA on ice for 30 mins. After heat shock at 42°C for 1 min, cells were incubated in 1 ml SOC media for rescue growth at 37 °C for 1 h. Finally, cells were plated on LB agar plates containing corresponding antibiotics.

### Pichia pastoris - CRISPR applications

Electrocompetent *P. pastoris* cells were freshly prepared for the transformation of pPpT6e-*cas9*-gRNA and HR-templates. A preculture of *P. pastoris* in 5 ml YPD medium was incubated for 9 h. Afterwards, the cells were transferred into 100 ml YPD medium with a starting OD₆₀₀ of 0.0025/ml and incubated at 30 °C until an OD₆₀₀ 1 - 2 was reached. Cells were harvested by centrifugation and treated with 8 ml LiAC-DTT solution (100 mM LiAC; 10 mM DTT; 0.6 M sorbitol; 10 mM TRIS-HCI pH 7.5) per 8 x 10⁸ cells. After incubation for 30 min, cells were centrifuged, resuspended in 1.5 ml cooled 1 M sorbitol (4 °C), centrifuged again and washed with 1.5 ml sorbitol. Finally, cells were resuspended at OD 10¹⁰ per ml in 1 M sorbitol (4 °C) and aliquoted for transformation. 1 µg pPpT6-*cas9* vector and 2 µg hybridized HR-template were co-transformed via electroporation in 80 µl freshly prepared competent cells. For rescue growth, cells were resuspended in 1 ml YPD:sorbitol 1:1, incubated for 1 h without shaking and afterwards 2 h at 250 rpm shaking at 30°C. Then, 120 - 200 µl cells were plated on YPD agar plates containing 200 µg/ml G418.

### Isolation of genomic DNA

Isolation of genomic DNA from *P. pastoris* was performed using MasterPure Yeast DNA Purification Kit (Epicentre) following manufacture's manual.

### Colony PCR

Colony PCR was performed using Q5 Polymerase Master Mix (NEB). Colonies were stamped on YPD agar plates and picked into the PCR solution. PCR program: (98°C 7 min, [98°C 10 sec, 72°C 10 sec, 72°C 30 s/kb]x30, 72°C 2 min).

### Activity assay

To measure enzymatic activity Phadebas Amylase Test (Magle Chemoswed AB) was used. Two Phadebas Amylase test tablets were resuspended in 40 ml Britton Robinson-Buffer (BR-Buffer: 40 mM Acetic acid; 40 mM boric acid; 40 mM phosphoric acid; pH adjusted with 10 M NaOH) *(*Britton, Hubert Thomas Stanley; Robinson, Robert Anthony (1931): CXCVIII.-Universal buffer solutions and the dissociation constant of veronal. In: J. Chem. Soc. 0 (0):1456-1462*. DOI: 10.1039*/*JR9310001456*.) and preheated on desired temperature in Eppendorf 96 well microtiterplates in a thermoshaker (BioShake IQ, AnalytikJena AG) at 1000 rpm for 10 min. 40 µl/well of enzyme sample were added and the reaction was performed for 20 min at 1500 rpm. The reaction was stopped by adding 60 µl/well 1 M NaOH and shaking. The solution was centrifuged for 5 min at 5100 rpm. 100 µl/well were analyzed for absorption at 620 nm to determine enzyme activities.

### Split-GFP assay

For quantification of protein amounts, the Split-GFP Assay was used (Cabantous, Stéphanie; Waldo, Geoffrey S. (2006): In vivo and in vitro protein solubility assays using split GFP. In: Nature methods 3 (10):845-854. DOI: 10.1038/nmeth932*;* Santos-Aberturas, Javier; Dörr, Mark; Waldo, Geoffrey S.; Bornscheuer, Uwe T. (2015): In-Depth High-Throughput Screening of Protein Engineering Libraries by Split-GFP Direct Crude Cell Extract Data Normalization. In: Chemistry & biology 22 (10):1406-1414. DOI: 10.1016/j.chembiol.2015.08.014). 10 µl undiluted amylase sample were treated with 90 µl thawed GFP1-10 solution in a 96 well assay plate (Corning 3904) and initial fluorescence was measured (485 nmₑₓ; 520 nmₑₘ). Fluorescence was measured after again after 16 h of incubation. GFP1-10 was purified after expression in *E. coli* BL21-GOLD(DE3).

Cells were freshly transformed with pET26b(+)-GFP1-10. 500 ml LB with Kanamycin (25 µg/ml) were inoculated with a 5 ml preculture, incubated for 2 h at 37 °C prior to induction with 1 mM β-D-1-thiogalactopyranoside (IPTG). Cells were incubated for 5 h at 37 °C and harvested by centrifugation at 10000 rpm for 10 min. Cell pellets were resuspended in 2 ml TNG-Buffer (100 mM Tris-HCI [pH 7.4], 100 mM NaCl, 10 % glycerol). Cell-disruption was conducted using sonication (50 % duty-cycle, output control 3) for 1 to 1.5 min and inclusion bodies were extracted by centrifugation at 10000 rpm for 15 min. The supernatant was discarded, and the resulting pellet was resuspended in 3 ml TNG-buffer. The sonication and centrifugation cycle was repeated twice more and the final pellet was resuspended in 1 ml 5 M Urea (50mM NaP-Puffer [pH 7.4]) per 75 g inclusion bodies. The solution was diluted in TNG-Buffer (25 ml per 1 ml urea), aliquoted and frozen at -20°C.

### SDS-PAGE

SDS-PAGE was performed using discontinuously gels (Invitrogen, NuPAGE 4-12% Bis-Tris-Gel) at 200 V for 40 min. Prior to loading on gel, samples were treated with appropriate amount of SDS sample buffer (Novex, NuPAGE LDS sample buffer 4x) and heated up to 95°C for 5 min.

### Results

### Identification of α-amylases

The amylase sequence of the thermostable α-amylase AM782 from *Rhizomucor pusillus* (US 7189552 B2) was used to identify homologous amylase sequences from the NCBI database via protein BLAST. AM782 was described as thermostable α-amylase with a high reaction speed. These characteristics of AM782 are highly desired in starch processing application. With a sequence identity of 67 % (99 % query cover) the sequence of the putative α-amylase LrAmy from *Lichtheimia ramose* was identified (Accession CDS12621.1). Furthermore, with a sequence identity of 59 % (92 % query cover) the described α-amylase RoAmy from *Rhizopus oryzae* was identified. RoAmy was successfully expressed in *Pichia pastoris,* characterized biochemically demonstrating that the enzyme shows acidic and thermostable activities (Li, Song; Zuo, Zhirui; Niu, Dandan; Singh, Suren; Permaul, Kugenthiren; Prior, Bernard A. et al. (2011). Gene cloning, heterologous expression, and characterization of a high maltose-producing α-amylase of Rhizopus oryzae. In: Applied biochemistry and biotechnology 164 (5): 581-592. DOI: 10.1007/s12010-011-9159-5).

### Markerless site-specific integration

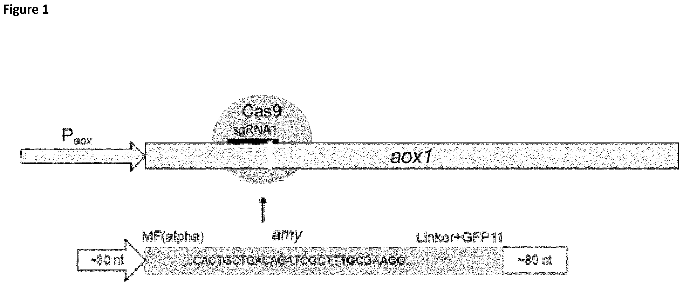

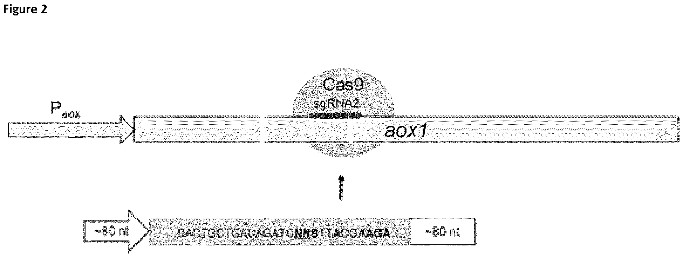

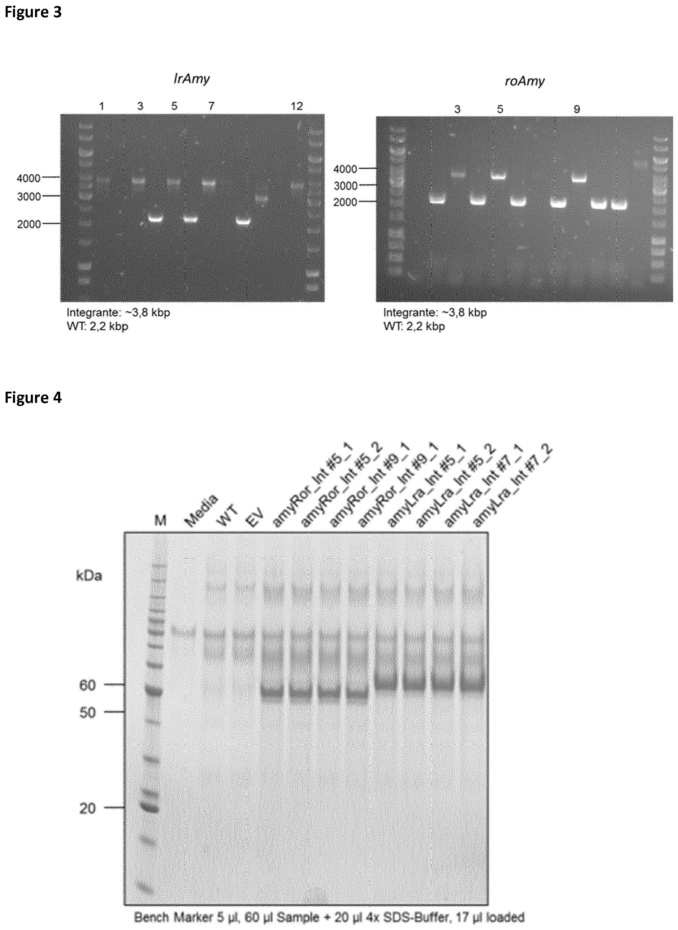

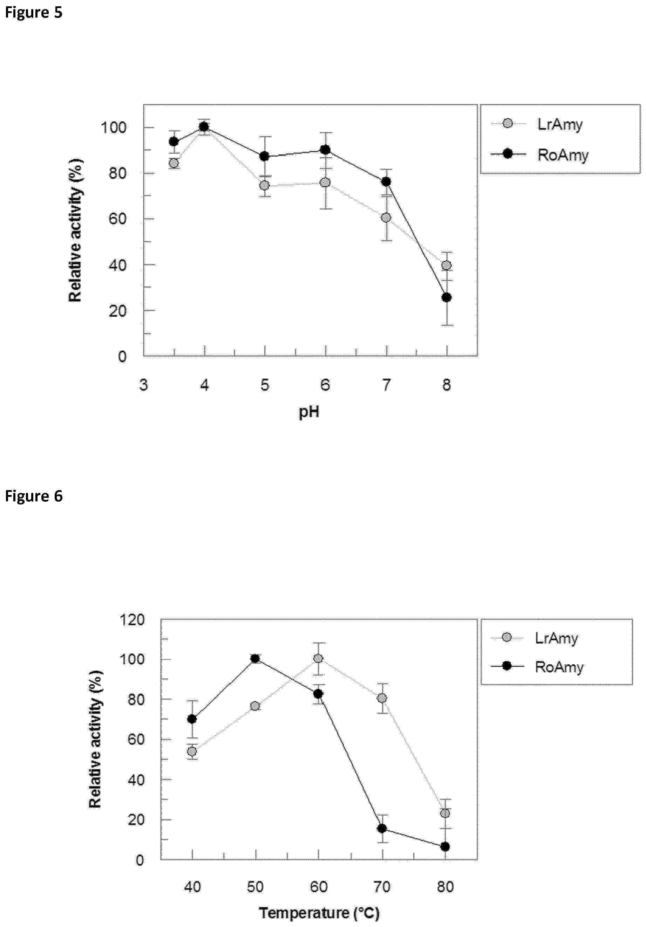

Single copies of the *lrAmy* and *roAmy* genes were integrated into the genome of *P. pastoris.* The amylase expression cassettes for the targeted integration consisted of homology arms of 92 bp at 5' position and 74 bp at 3' position (Figure 1). Later, for the protein engineering approach the homology template contained specific mutations (Figure 2). The *aox1* locus was chosen as integration site in order to use the strong and inducible P*ₐₒₓ₁* promotor for gene expression. The gene cassettes were amplified from pPpT4-alphaS-*roAmy-gfp11* and pPpT4-alphaS*-lrAmy-gfp11* by the primers Amy_Int_FW/Amy_Int_RV. The amplified products were co-transformed with pPpT6e-*cas9*-sgRNA1 and plated on YPD agar plates containing G418 (200 µg/ml). Colony PCRs and subsequent sequencing of the amplified DNA fragments verified the correct integration of the expression cassette with an integration rate of about 50 % (Figure 3). The resulting strains *P. pastoris* LrAmy_Int and RoAmy_Int were cured from the pPpT6e-*cas9* vector by cultivation in YPD medium without antibiotics. 24 colonies were picked and replica plated on YPD agar plates supplemented with G418 (200 µg/ml) to identify strains lacking the pPpT6e-*cas9* vector. Expression studies were performed in M2P-labs medium. SDS-PAGE analysis of culture supernatants shows the secretion of both amylases (Figure 4). LrAmy runs at 55 kDa and RoAmy at 50 kDA. Both amylases were characterized biochemically for their temperature and pH profiles using the Phadebas assay. Both amylases show pH optima at pH 4 (Figure 5). The temperature optimum lies at 50 °C for RoAmy and at 60 °C for LrAmy (Figure 6).

### Testing site specific mutations

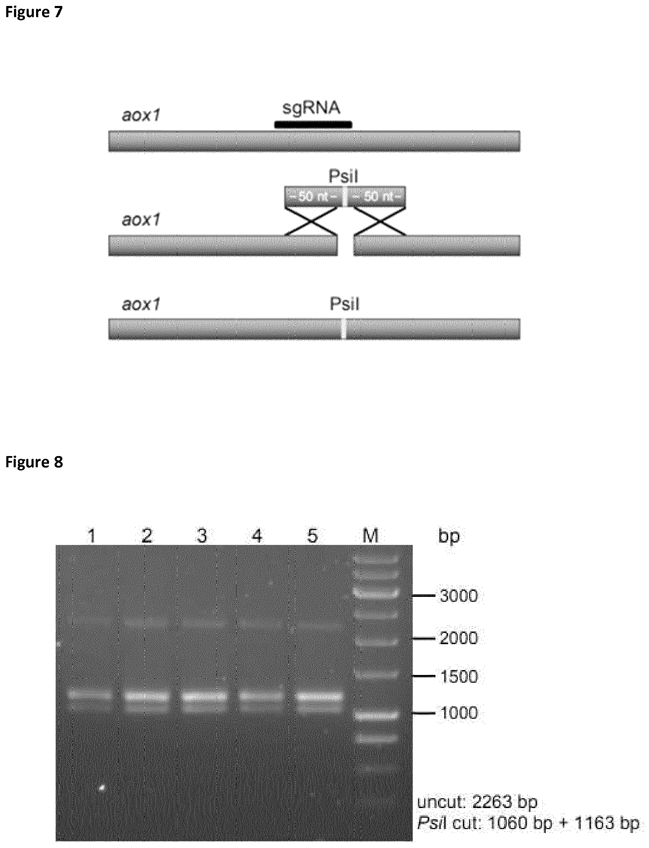

To test the efficiency for integration of short donor DNA into a *P. pastoris* WT strain with the use of the CRISPR-Cas9 technology, the *aox1* locus was targeted with a 100 nt HR-template and corresponding sgRNA (SEQ ID Nos. 9-12). This template was cotransformed with the pPpT6e-*cas9*-sgRNA1 vector. The double-stranded donor DNA contained a recognition site of the endonuclease *Psi*I*.* After gene integration, the genes were amplified by PCR. The resulting PCR products were digested with endonuclease and the *Psi*I site was used to identify correct integrands (Figure 7). Sequencing of the amplified fragments also confirmed the presence of the *Psi*I recognition site. All analyzed CFUs showed the expected cleavage products (Figure 8) and the successful recombination could be confirmed by Sanger sequencing, demonstrating an integration rate of up to 100 %.

### Protein Engineering

The 3DM database for α-amylases was used for site selection. LrAmy is grouped into subfamily 2AAAA and RoAmy is grouped into 2GUYA based on their structures. For hot spot identification, the focus lied on amino acids that are important for ligand binding to improve specific activities and amino acids that show high flexibility (b-value) to improve thermostability. TopLib was used to calculate the number of colonies that have to be analyzed in order to catch one of the two best variants with a probability of 98.8 % (Nov, Yuval (2012): When second best is good enough. Another probabilistic look at saturation mutagenesis. In: Applied and environmental microbiology 78 (1):258-262. DOI: 10.1128/AEM.06265-11).

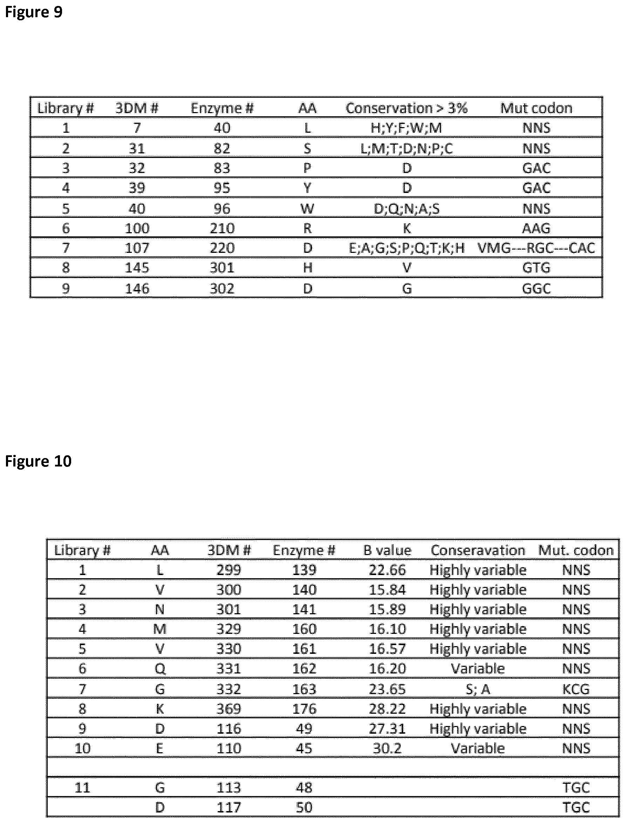

For LrAmy, nine amino acid positions were mutated to improve the specific activity (Figure 9). Sites showing low conservation were mutated via NNS degenerative codons (positions 40, 82 and 96), whereas 5 positions were specifically exchanged (P83D, Y95D, R210K, H301V and D302G). Codons for specific amino acids combinations were calculated using the software DC-analyzer (Wang, Xiong; Zheng, Kai; Zheng, Huayu; Nie, Hongli; Yang, Zujun; Tang, Lixia (2014): DC-Analyzer-facilitated combinatorial strategy for rapid directed evolution of functional enzymes with multiple mutagenesis sites. In: Journal of biotechnology 192 Pt A: 102-107. DOI: 10.1016/j.jbiotec.2014.10.023). For 3DM site 220, a combination of three degenerate codons was used for amino acid exchanges of 9 amino acids only. HR-templates containing degenerate codons for mutagenesis were mixed in specific ratios (5:2:1 VMG:RGC:CAC). *P. pastoris* was cotransformed with the HR-templates containing the codons for mutagenesis (some random examples herefore are SEQ ID Nos. 17-24, which should not be restrictive in any way) and the pPpT6e-*cas9* plasmid containing the relevant sgRNA. Some random examples of these sgRNAs are SEQ ID Nos. 13-16, which also should not be seen as restrictive for the present invention.

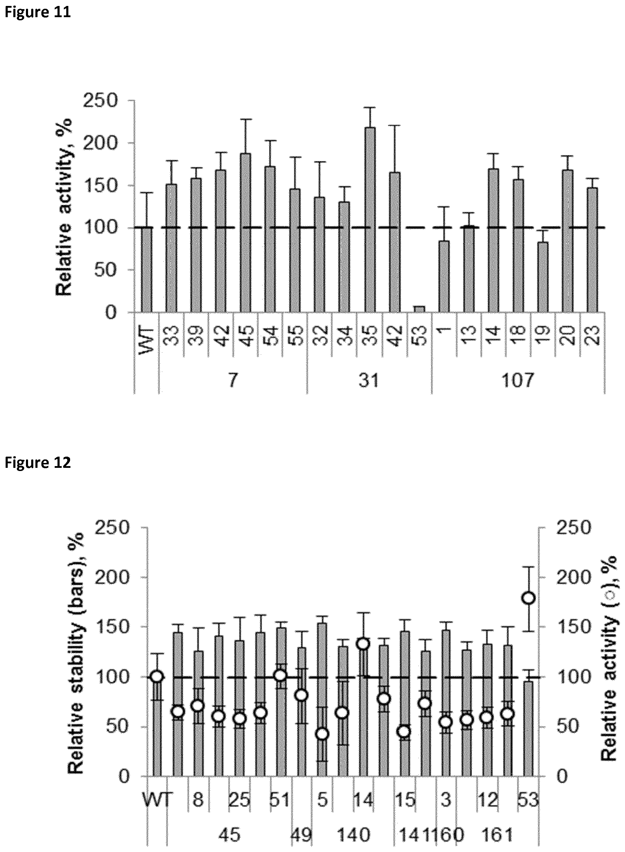

The mutant libraries were expressed and screened for improved specific activities using the Phadebas assay (activity) and the Split-GFP assay (protein amount). In 14 cases, an improved specific activity was demonstrated. In total, 6, 4 and 4 mutants comprising positions 40, 82 and 220, respectively, were identified that show significantly higher specific activity (130 - 219 % of WT activity) compared to the WT (Figure 11).

For RoAmy, 10 sites with the highest b-value in a structure model were identified for the improvement of its thermostability (Figure 10). All sites, except Q332 were mutated randomly with NNS codons. For Q332 a degenerate codon with sequence KCG was chosen. In addition, two cysteins were integrated at G113 and D117 to introduce a disulfide bridge. It was previously shown that this leads to improved thermostability in a homologous enzyme *(*Liu, Hsuan-Liang; Wang, Wen-Chi (2003): Protein engineering to improve the thermostability of glucoamylase from Aspergillus awamori based on molecular dynamics simulations. In: Protein Engineering, Design and Selection 16 (1):19-25). *P. pastoris* was transformed with the HR-templates that contain the codons for mutagenesis together with pPpT6e-*cas9* containing relevant sgRNA. Samples were heated at 60°C for 1 h and residual activity was measured to identify mutants with improved thermostability. The incubation time is adapted to WT RoAmy with 30 % residual activity. The mutant libraries were expressed and screened for improved amylase stability. The Split-GFP assay for protein quantification was also performed to assess both the relative amylase activity and to ensure that the mutants were sufficiently active. 21 mutants with improved stability were identified. In total, 6,1, 4, 2,1 and 3 mutants comprising positions 110, 116, 300, 301, 329 and 330, respectively, were identified that show significantly improved thermostabilities at 60 °C of 126 - 154 % compared to the WT.

### Sequencing of mutations

Two mutated strains, as representatives for the mutant library, were sequenced in order to analyze the HR efficiency in the performed mutagenesis. All transformants of one mutant library were pooled from an agar plate and total gDNA was isolated. Standard PCR was performed on the pooled gDNA in order to analyze the amylase genes via Sanger sequencing. LrAmy_P83 was chosen as representatives for specific amino acid exchanges and LrAmy_D220 as representatives for amino acid exchanges using degenerate codons. Sequence alignments of LrAmy_P83 and LrAmy_D220 show successful integration of the applied HR-templates without any apparent non recombination background (Fig. 13). In case of LrAmy_D220 the codon site shows overlapping signals, which demonstrated the successful integration of several codons using the degenerate codon templates. Especially, the silent mutations that were introduced in the Cas9 seed region in all mutants, independently from the mutations to improve the amylases, were analyzed. The integration efficiency was confirmed by a 100 % mutation efficiency of the PAM region to suppress double strand breaks after HR.

## Claims

1. A method for engineering a protein in a host cell, comprising the following steps:
identifying a protein of interest and introducing the coding sequence of the protein of interest into the genome of the host cell;
screening for hotspots for amino acid mutations in the protein of interest;
generating a set of specific guide RNAs and libraries of homologous recombination template that generate mutations at the desired sites within the protein coding region;
introducing the guide RNA and the library of homologous recombination template into the host cell, thereby producing mutated protein coding regions;
screening to select for cells that express the protein of interest with desired activity and/or property from the mutated protein coding regions, thereby providing an engineered protein.

2. The method of claim 1, wherein the coding sequence of the protein of interest is introduced into the genome of the host cell by means of a CRISPR-Cas-system.

3. The method of claim 2, wherein the CRISPR-Cas system is a Class II CRISPR-Cas-system.

4. The method of one or more of the preceding claims, wherein the host is a prokaryotic host.

5. The method of one or more of the preceding claims, wherein the host is an eukaryotic host.

6. The method of one or more of the preceding claims, wherein the protein of interest is a hydrolase.

7. The method of one or more of the preceding claims, wherein the protein of interest is an amylase.

8. The method of claim 7, wherein the amylase is an α-amylase.

9. The method of claim 8, wherein the α-amylase is AmyLra and/or RoAmy.

10. The method of one or more of the preceding claims, wherein the protein coding region is *aox1.*

11. The method of one or more of the preceding claims, wherein Cas9 is used as an endonuclease.

12. The method of one or more of the preceding claims, wherein the guide RNA is selected from the group consisting of SEQ ID Nos. 9, 10, 13-16.

13. The method of one or more of the preceding claims, wherein the homologous recombination template is selected from the group consisting of SEQ ID No. 11, 12, 17-24.


SEQUENCE LISTING

<110>  B.R.A.I.N. AG

<120>  A method for engineering a protein

<130>  C 3653 EP

<160>  24

<170>  PatentIn version 3.5

<210>  1
<211>  49
<212>  DNA
<213>  Unknown

<220>
<223>  Artificial Sequence

<400>  1
gtctctctcg agaagagaga ggccgaagct atgcgtccgc tggttaaac                49


<210>  2
<211>  52
<212>  DNA
<213>  Unknown

<220>
<223>  Artificial Sequence

<400>  2
ggcattctga catcctcttg agcggcctta tgtaatccca gcagcattta cg            52


<210>  3
<211>  51
<212>  DNA
<213>  Unknown

<220>
<223>  Artificial Sequence

<400>  3
gtctctctcg agaagagaga ggccgaagct atgagcgatt gggaaaatcg c             51


<210>  4
<211>  46
<212>  DNA
<213>  Unknown

<220>
<223>  Artificial Sequence

<400>  4
ctgcgcgttt aaccagcgga cgcatagctt cggcctctct cttctc                   46


<210>  5
<211>  52
<212>  DNA

<213>  Unknown

<220>
<223>  Artificial Sequence

<400>  5
cgtaaatgct gctgggatta cataaggccg ctcaagagga tgtcagaatg cc          52


<210>  6
<211>  42
<212>  DNA
<213>  Unknown

<220>
<223>  Artificial Sequence

<400>  6
gcgattttcc caatcgctca tagcttcggc ctctctcttc tc          42


<210>  7
<211>  51
<212>  DNA
<213>  Unknown

<220>
<223>  Artificial Sequence

<400>  7
ggttccaatt gacaagcttt tgattttaac gacttttaac gacaacttga g          51


<210>  8
<211>  99
<212>  DNA
<213>  Unknown

<220>
<223>  Artificial Sequence

<400>  8
cgataagacc aactttcaag gagtggtcca agtttgccaa tcttccggca atacaggatc          60

cactggatcc accattatgt aatcccagca gcatttacg          99


<210>  9
<211>  67
<212>  DNA
<213>  Unknown

<220>
<223>  Artificial Sequence

<400>  9
gatcgatatc ctgatgagtc cgtgaggacg aaacgagtaa gctcgtcgat atcctagttc          60

taggtgg          67


<210>  10
<211>  67

<212>  DNA
<213>  Unknown

<220>
<223>  Artificial Sequence

<400>  10
aaacccacct agaactagga tatcgacgag cttactcgtt tcgtcctcac ggactcatca      60

ggatatc                                                                67


<210>  11
<211>  100
<212>  DNA
<213>  Unknown

<220>
<223>  Artificial Sequence

<400>  11
ctaattattc gaaacgatgg ctatccccga agagtttgat atcctagttc taggtttata      60

atccagtgga tcctgtattg ccggaagatt ggcaaacttg                          100


<210>  12
<211>  100
<212>  DNA
<213>  Unknown

<220>
<223>  Artificial Sequence

<400>  12
caagtttgcc aatcttccgg caatacagga tccactggat tataaaccta gaactaggat      60

atcaaactct tcggggatag ccatcgtttc gaataattag                          100


<210>  13
<211>  67
<212>  DNA
<213>  Unknown

<220>
<223>  Artificial Sequence

<400>  13
gatcagcagg ctgatgagtc cgtgaggacg aaacgagtaa gctcgtccct gctgcatttg      60

tcggaat                                                                67


<210>  14
<211>  67
<212>  DNA
<213>  Unknown

<220>
<223>  Artificial Sequence

<400>  14

aaacattccg acaaatgcag cagggacgag cttactcgtt tcgtcctcac ggactcatca 60

gcctgct 67

<210> 15
<211> 67
<212> DNA
<213> Unknown

<220>
<223> Artificial Sequence

<400> 15
gatcaccgta ctgatgagtc cgtgaggacg aaacgagtaa gctcgtctac ggttaaacat 60

gtcagac 67

<210> 16
<211> 67
<212> DNA
<213> Unknown

<220>
<223> Artificial Sequence

<400> 16
aaacgtctga catgtttaac cgtagacgag cttactcgtt tcgtcctcac ggactcatca 60

gtacggt 67

<210> 17
<211> 90
<212> DNA
<213> Unknown

<220>
<223> Artificial Sequence

<400> 17
gattacattc aaggcatggg ctttgatgca atttggattt cagacatccc gacaaatgca 60

gcaggcggat atcatggcta ttgggcgaca 90

<210> 18
<211> 90
<212> DNA
<213> Unknown

<220>
<223> Artificial Sequence

<400> 18
tgtcgcccaa tagccatgat atccgcctgc tgcatttgtc gggatgtctg aaatccaaat 60

tgcatcaaag cccatgcctt gaatgtaatc 90

<210> 19
<211> 90

<212>  DNA
<213>  Unknown

<220>
<223>  Artificial Sequence

<400>  19
atggatttga tggcattaga atcgatacgg ttaaacatgt gcgtcagvmg ttttgggacg      60

gctataacaa agcggcaggc gtttttgcaa                                      90


<210>  20
<211>  90
<212>  DNA
<213>  Unknown

<220>
<223>  Artificial Sequence

<400>  20
ttgcaaaaac gcctgccgct ttgttatagc cgtcccaaaa ckbctgacgc acatgtttaa      60

ccgtatcgat tctaatgcca tcaaatccat                                      90


<210>  21
<211>  90
<212>  DNA
<213>  Unknown

<220>
<223>  Artificial Sequence

<400>  21
atggatttga tggcattaga atcgatacgg ttaaacatgt gcgtcagrgc ttttgggacg      60

gctataacaa agcggcaggc gtttttgcaa                                      90


<210>  22
<211>  90
<212>  DNA
<213>  Unknown

<220>
<223>  Artificial Sequence

<400>  22
ttgcaaaaac gcctgccgct ttgttatagc cgtcccaaaa gcyctgacgc acatgtttaa      60

ccgtatcgat tctaatgcca tcaaatccat                                      90


<210>  23
<211>  90
<212>  DNA
<213>  Unknown

<220>
<223>  Artificial Sequence

<400>  23

atggatttga tggcattaga atcgatacgg ttaaacatgt gcgtcagcac ttttgggacg 60

gctataacaa agcggcaggc gtttttgcaa 90

<210> 24
<211> 90
<212> DNA
<213> Unknown

<220>
<223> Artificial Sequence

<400> 24
ttgcaaaaac gcctgccgct ttgttatagc cgtcccaaaa gtgctgacgc acatgtttaa 60

ccgtatcgat tctaatgcca tcaaatccat 90